# DEMANDE DE BREVET EUROPEEN

(11) **EP 3 604 270 A1**
(43) Date de publication de la demande: **05.02.2020**
(21) Numéro de dépôt: 19199227.0
(22) Date de dépôt: 28.09.2016
(51) Int. Cl.: C07C 227/06, C08G 69/08

(54) **COMPOSITION A BASE D'AMINO ACIDE OU ESTER DE QUALITÉ POLYMÈRE ET PROCÉDÉS D'OBTENTION**

(30) Priorité: 30.09.2015 FR 1559260
(62) Demande divisionnaire de: 16785240.9
(71) Demandeur: ARKEMA FRANCE, 92700 Colombes (FR)
(72) Inventeur: COUTURIER, Jean-Luc, 69493 PIERRE-BENITE CEDEX (FR); DEVAUX, Jean-François, 69493 PIERRE-BENITE CEDEX (FR); DUBOIS, Jean-Luc, 92705 COLOMBES CEDEX (FR); BRIFFAUD, Thierry, 27470 SERQUIGNY (FR)
(74) Mandataire: Kling, Simone

(57) **Abrégé**

La présente invention a pour objet une composition comprenant au moins un ester ou acide α,ω-amino-alcanoïque et au moins un acide ou ester alcanoïque, caractérisée en ce que :
- l'ester ou acide α,ω-amino-alcanoïque a pour formule H₂N-(CH₂)ₙ-COOR avec n=9 à 13 et R est un groupement alkyle ou un hydrogène ,
- l'acide ou ester alcanoïque a pour formule R₁-COOR₂ avec R₁ un groupement alkyle linéaire ou ramifié de formule CₘH₂ₘ₊₁ ou CₘH₂ₘ₋₁ ou CₘH₂ₘ₋₃ où m=6 à 20 et R₂ est un groupement alkyle ou un hydrogène,
- le ratio molaire entre acide(s) ou ester(s) alcanoïques et ester(s) ou acide(s) α,ω-amino-alcanoïque est compris dans la gamme de 0,001 à 0,4%.

La présente invention a également pour objet de nouveaux procédés d'obtention d'une telle composition.

## Description

### DOMAINE DE L'INVENTION

L'invention a pour objet une composition à base d'acide ou d'ester α,ω-amino-alcanoïque à chaine longue et d'acide ou d'ester alcanoïque, et les procédés d'obtention de cette composition. Cette composition selon l'invention est utilisable en particulier comme matière première pour la fabrication de polyamides ou copolyamides.

### ARRIERE PLAN TECHNIQUE

L'industrie des polyamides utilise toute une gamme de monomères formés à partir de diamines et de diacides, de lactames, et surtout à partir d' α,ω-aminoacides. Ces derniers sont définis par la longueur de chaîne méthylène (-CH2)n séparant deux fonctions amide -CO-NH-.

Par acides ou esters α,ω-aminoalcanoïques, ci-après simplement « aminoacides ou aminoesters », au sens de l'invention, on entend en fait tout α,ω-aminoacide à chaîne longue, c'est-à-dire dont la chaîne comporte au moins 8 atomes de carbones.

En effet, les polyamides visés par la présente invention sont des polyamides techniques, c'est-à-dire des polyamides de performance, de haute performance, voire de très haute performance, fabriqués à partir de monomères comportant au moins 8 atomes de carbone, de préférence au moins 10 atomes de carbones; par opposition aux polyamides dits de « commodité », tels que le « nylon 6 », dont les quantités (volumes) commercialisées sont très supérieures et les coûts très inférieurs à ceux des polyamides techniques.

La polymérisation à partir de monomères d'acide ou d'ester α,ω-amino-alcanoïque longue chaine, pour atteindre des degrés de polymérisation élevés (supérieurs à 80), requiert des monomères de grande pureté.

Parmi les procédés les plus prometteurs de fabrication d'acide ou d'ester α,ω-amino-alcanoïque longue chaine, on peut citer des procédés d'hydrogénation de nitriles acides insaturés ou nitriles esters insaturés tels que ceux décrits dans les demandes de brevet WO08104722, WO14122412. Ces demandes de brevet décrivent un procédé de synthèse d'aminoacide par métathèse, hydrolyse puis hydrogénation.

Lors des essais de fabrication de polyamides à partir de ces acides ou esters α,ω-amino-alcanoïque longue chaine, deux problèmes ont été mis en évidence. Dans certains cas, il n'est pas possible d'obtenir des polyamides avec un degré de polymérisation élevé. Dans d'autres cas, la rhéologie à l'état fondu du polyamide obtenu est instable à haute température, de l'ordre de 200 à 350°C par exemple : la viscosité à l'état fondu tend à augmenter. Cette instabilité peut entrainer, au cours de la transformation des granulés en pièces moulées ou extrudées (et en particulier lors d'un arrêt machine imprévu), un blocage des vis d'injection ou d'extrusion, dû à la viscosité trop élevée atteinte. Ce blocage présente plusieurs inconvénients : Il est nécessaire de démonter la machine et de la nettoyer avant redémarrage, ce qui entraîne une perte de temps de production, voire même une détérioration du matériel.

Il existe donc un réel besoin de trouver des matières premières à base d'acide ou d'ester α,ω-amino-alcanoïque longue chaine qui permettent de produire des polyamides longue chaine de degré de polymérisation élevé, et qui sont stables à haute température, c'est-à-dire dont la viscosité à l'état fondu n'augmente pas.

La Demanderesse a maintenant trouvé une composition à base d'acide ou d'ester α,ω-amino-alcanoïque longue chaine, qui permet d'atteindre ces deux buts, en combinant l'acide ou l'ester α,ω-amino-alcanoïque avec une teneur particulière en acide ou ester alcanoïque. Ce dernier a en effet été identifié par les Inventeurs comme un élément clé de la composition pour produire des polyamides longue chaine à degré de polymérisation élevée, et stables à haute température.

### DESCRIPTION DE L'INVENTION

Tout au long du texte, les pressions sont exprimées en bars ou mbars absolus.

La présente invention a donc pour objet une composition comprenant au moins un ester ou acide α,ω-amino-alcanoïque et au moins un acide ou ester alcanoïque, caractérisée en ce que :
- l'ester ou acide α,ω-amino-alcanoïque a pour formule H2N-(CH2)n-COOR avec n=9 à 13 et R est un groupement alkyle ou un hydrogène,
- l'acide ou ester alcanoïque a pour formule R1-COOR2 avec R1 un groupement alkyle linéaire ou ramifié de formule CmH2m+1 ou CmH2m-1 ou CmH2m-3 où m=6 à 20 et R2 est un groupement alkyle ou un hydrogène ,
- le ratio molaire entre acide(s) ou ester(s) alcanoïques et ester(s) ou acide(s) α,ω-amino-alcanoïque est compris dans la gamme de 0,001 et 0,4% et de préférence entre 0,01 et 0,2% et plus préférentiellement entre 0,02 et 0,1%.

De manière avantageuse, n=10 ou 11, et R est un groupement méthyle ou éthyle ou butyle ou un hydrogène.

De préférence, R1 est un groupement alkyle linéaire de composition Cn-1H2n-1 et R2 est un groupement méthyle ou éthyle ou un hydrogène.

Avantageusement, la composition selon l'invention comprend en outre au moins un nitrile acide ou un nitrile ester de formule NC-(CH2)(n-1)-COOR, dans laquelle le ratio molaire de nitrile par rapport à l'ester ou acide α,ω-amino-alcanoïque est compris dans la gamme de 0,0001% à 0,5%, de préférence compris dans la gamme de 0,0001% à 0,1% , de préférence compris dans la gamme de 0,0001% à 0,05%. Avantageusement, la composition selon l'invention comprend en outre au moins une amine secondaire de formule ROOC-(CH₂)ₙ-NH-(CH₂)ₙ-COOR, dans laquelle le ratio molaire par rapport à l'ester ou acide α,ω-amino-alcanoïque est compris dans la gamme de 0,0001% à 1%, de préférence de 0,0001% à 0,5%. Avantageusement, la composition selon l'invention comprend en outre au moins un dimère ROOC-(CH2)n-NH-CO-(CH2)n-NH2, le ratio molaire en dimère par rapport à l'ester ou acide α,ω-amino-alcanoïque étant compris dans la gamme de 0,0001% à 5% et de préférence compris dans la gamme de 0,0001% à 1%. Avantageusement, la composition selon l'invention comprend en outre au moins un composé choisi parmi : les composés inertes vis-à-vis de la polymérisation, les alcools, l'eau, et leurs mélanges.

Les composés inertes vis-à-vis de la polymérisation sont avantageusement choisis parmi : les hydrocarbures aromatiques, tels que le benzène, toluène, xylène, l'éthylbenzène ; les hydrocarbures aliphatiques, tels que le cyclohexane, le méthylcyclohexane, le pentane, l'heptane ; ou une coupe aliphatique ; ou un éther tel le tétrahydrofurane, diéthyléther, diisopropyl éther, le méthyltertiobutyléther ; et leurs mélanges.

Les alcools sont avantageusement choisis parmi le méthanol, l'éthanol, le butanol, et leurs mélanges.

De préférence, la teneur éventuelle en composés inertes, en alcool et/ou en eau de la présente composition, est inférieure à 90%, de préférence inférieure à 50%, de préférence inférieure à 10%, et plus préférablement inférieure à 2%, en poids sur le poids total de la composition.

Avantageusement, la teneur en ester ou acide α,ω-amino-alcanoïque est supérieure à 10%, de préférence supérieure à 50%, de préférence supérieure à 90% et plus préférablement supérieure à 98%, en poids sur le poids total de la composition.

La présente invention a également pour objet un polymère, notamment polyamide, obtenu par polymérisation à partir de la composition selon l'invention.

La présente invention décrit également deux nouveaux procédés possibles (ci-après procédé A et procédé B) de préparation d'une telle composition d'ester α,ω-amino-alcanoïque longue chaine et d'acide ou ester alcanoïque dont le ratio molaire entre l'acide ou ester alcanoïque et l'ester α,ω-amino-alcanoïque longue chaine est compris dans la gamme de 0,001 à 0,4% à partir de nitrile ester insaturé. Tout l'enjeu technique de ces procédés est de réussir à limiter la teneur en acide / ester alcanoïque dans la composition à une teneur inférieure à 0,4% et d'obtenir un bon rendement chimique, supérieur à 70%, de préférence supérieur à 80% et de préférence supérieur à 90%.

### PROCEDES DE PREPARATION DE LA COMPOSITION

### Procédé A

Selon un premier mode de réalisation, la composition selon l'invention est produite par une réaction d'hydrogénation à partir d'un nitrile acide aliphatique insaturé ou d'un nitrile ester aliphatique insaturé tel que :
- le nitrile acide ou ester aliphatique insaturé contient moins de 0,3% d'acide ou ester alcanoïque, de préférence de 0,001 à 0,3%, et de préférence moins de 0,1%, de préférence de 0,001 à 0,1% d'acide/ester alcanoïque;
- l'hydrogénation est réalisée en présence d'un catalyseur hétérogène qui comprend au moins du Co et/ou du Ru ;
- l'hydrogénation est réalisée en présence d'une base.

De façon préférentielle, le procédé comprend en outre une étape de séparation de lourds sur appareil à cours temps de séjour.

### Procédé B

Selon un deuxième mode de réalisation, la composition selon l'invention peut aussi être produite par un enchainement de deux étapes d'hydrogénation à partir d'un nitrile acide aliphatique insaturé ou d'un nitrile ester aliphatique insaturé tel que :
- le nitrile acide ou ester aliphatique insaturé contient comme impureté moins de 0,3% d'acide ou ester alcanoïque, de préférence de 0,001 à 0,3% et de préférence moins de 0,1%, de préférence de 0,001 à 0,1% d'acide/ester alcanoïque.
- la première étape d'hydrogénation a pour rôle de transformer partiellement ou totalement le nitrile acide aliphatique insaturé ou le nitrile ester aliphatique insaturé en nitrile ou ester aliphatique saturé et est réalisée en présence d'un catalyseur hétérogène comprenant au moins du palladium et/ou du rhodium et/ou du platine.
- la deuxième étape a pour objet de former l'amino acide ou ester saturé et est réalisée sur un catalyseur hétérogène qui comprend au moins du Ni et/ou du Co et/ou du Ru

De façon préférentielle, le procédé comprend en outre une étape de séparation de lourds sur appareil à cours temps de séjour.

Dans les deux cas de procédé, le nitrile ester/acide aliphatique insaturé est de formule :

NC-(CH2)p-CH=CH-(CH2)q-CO2R

Avec p>=0 et q>=0 et p+q+3=n défini précédemment
R défini comme précédemment.

La double liaison est indifféremment cis ou trans. De préférence, il s'agit d'un mélange cis et trans.

Il peut être produit par métathèse, notamment selon les procédés décrits dans : WO2008/104722, WO10/089512, WO13/017782, WO13/017786, WO13/030481, WO14/106766, WO14/106723, WO14/106724, WO14/147337.

De préférence, un mélange brut issu de la réaction de métathèse est soumis à des étapes de purifications. Ces purifications sont par exemple des étapes pour éliminer ou inactiver le catalyseur de métathèse, des étapes de distillation pour éliminer le solvant et/ou des réactifs ou impuretés légères, et/ou des impuretés lourdes. Les réactifs éliminés sont par exemple des esters ou des acides ou des nitriles. Les impuretés lourdes éliminées peuvent être par exemple des acides ou esters gras tel l'oléate de méthyle ou des diesters tel le composé RO2C-(CH2)q-CH=CH-(CH2)q-CO2R.

Le (mélange ainsi obtenu à base de) nitrile ester aliphatique insaturé ou le nitrile acide aliphatique insaturé utilisé contient moins de 0,3% d'acide ou ester alcanoïque et de préférence moins de 0,1%.

De préférence, le nitrile ester aliphatique insaturé ou le nitrile acide aliphatique insaturé contient moins de 1% et de préférence moins de 0,1% de diester.

Caractéristiques spécifiques au procédé A :
L'hydrogénation est réalisée en présence d'au moins un catalyseur hétérogène d'hydrogénation qui contient du cobalt et/ou du ruthénium.

Le catalyseur selon l'invention contient typiquement 0,1 à 10% de ruthénium et de préférence 1 à 5%, et/ou 1% à 90% de cobalt et de préférence 5% à 60%.

Le cobalt et/ou le ruthénium est de préférence dans sa forme réduite métallique. Le catalyseur introduit dans les réacteurs selon l'invention peut néanmoins contenir des oxydes de ruthénium ou de cobalt par exemple sous la forme d'une couche de passivation.

En plus du cobalt ou ruthénium, le catalyseur peut contenir d'autres métaux, tels que le fer, le nickel, le chrome, le manganèse, le rhodium, l'osmium, l'iridium, le platine, ou le palladium. Le catalyseur peut également être dopé par des alcalins tels que le sodium, potassium, rubidium ou césium ou des alcalinoterreux tels que le magnésium, le calcium, le strontium ou le baryum.

Le catalyseur peut se trouver à l'état finement divisé, par exemple sous la forme d'un catalyseur de type Raney. Il peut également se trouver sous la forme de granules, ou encore peut être déposé sur un support. Des exemples de supports appropriés sont la pierre ponce, le dioxyde de titane, le carbone, le charbon, le carbure de silicium (de préférence de forme beta SiC), l'alumine, la silice, un mélange de silice et d'alumine, la stéatite.

Le catalyseur sous forme de granule ou déposé sur un support peut se présenter sous une forme quelconque telle que : sphère, grain, cylindre creux, trilobe et quadrilobe, ainsi que sous la forme de cylindres, extrudés ou compressés éventuellement en utilisant un agent de pastillage.

La préparation des catalyseurs finement divisés de type Raney est amplement connue dans l'art antérieur.

Les catalyseurs peuvent être préparés avec les méthodes connues pour le chargement des métaux sur un support. Par exemple, un catalyseur peut être préparé en mettant en contact le support avec une solution aqueuse ou alcoolique de trichlorure de ruthénium, nitrosylnitrate de ruthénium, ou tout sel de ruthénium soluble dans l'eau ou un alcool, de carbonate de cobalt, nitrate de cobalt ou tout sel de cobalt soluble dans l'eau ou l'alcool, puis en évaporant l'eau ou l'alcool et en activant le catalyseur par des moyens de chauffage et de réduction dans un courant gazeux contenant de l'hydrogène. Les catalyseurs contenant du cobalt seront calcinés typiquement à des températures de 300 à 500°C et réduits à des températures de 300 à 500°C. Les catalyseurs au ruthénium seront calcinés typiquement à des températures de 200 à 400°C et réduits à des températures de 100 à 400°C.

Les autres métaux tels que le fer, le nickel, le chrome, le manganèse, le rhodium, l'osmium, l'iridium, le platine, ou le palladium, ainsi que les alcalins ou alcalino-terreux peuvent être introduits sur le catalyseur de la même façon que le ruthénium ou le cobalt, soit préalablement à la mise en contact de la solution de sel de ruthénium ou de cobalt avec le support, soit de façon concomitante, soit dans une étape postérieure.

Les catalyseurs peuvent également être préparés par des techniques connues de précipitation ou coprécipitation suivies d'étapes de calcination et de réduction dans un courant gazeux contenant de l'hydrogène. Les étapes de précipitation permettent d'obtenir un solide contenant du cobalt principalement sous forme de CoO, Co3O4 et /ou CoO(OH), éventuellement dopés par du fer, nickel, chrome, manganèse, rhodium, osmium, iridium, platine, ou palladium, ou des alcalins ou alcalino-terreux ou de la silice ou de l'alumine ou un mélange de silice et d'alumine. Le solide peut subir une calcination à une température typique de 300 à 500°C et il est réduit dans un courant gazeux contenant de l'hydrogène à des températures de 300 à 500°C. Les catalyseurs utilisés peuvent être sensibles à l'air, voire pyrophoriques. Ils peuvent subir une étape de passivation de façon à pouvoir charger les réacteurs d'hydrogénation sans risque. Il existe de nombreuses techniques de passivation. Par exemple, ils peuvent être passivés à l'air appauvri ou à l'air à une température proche de la température ambiante, ou bien avec des composés soufrés. Ils peuvent être encapsulés dans des paraffines ou des esters saturés qui vont fondre au démarrage du réacteur et s'éliminer dans le milieu réactionnel. Ils peuvent aussi être conditionnés et manipulés en présence d'eau ou d'un solvant organique. Avantageusement, la réaction d'hydrogénation est conduite à une pression comprise dans la gamme de 5 à 100 bars, de préférence de 20 à 80 bars. Avantageusement, la température est comprise dans la gamme de 50 à 150°C, de préférence de 80 à 110°C.

On ajoute une base pour diminuer la sélectivité en amine secondaire. La base peut être typiquement la soude, le méthoxyde de sodium, la potasse, le tertiobutylate de potassium ou l'ammoniac, et leurs mélanges. De préférence, on utilise de l'ammoniac. Le ratio molaire base/nitrile ester insaturé est typiquement compris dans la gamme de 0,1 à 4 et de préférence de 0,3 à 2,5.

Selon un mode de réalisation, on réalise l'hydrogénation en absence de solvant.

Selon un mode de réalisation préféré, l'hydrogénation est réalisée en présence d'un solvant. Le solvant peut être un hydrocarbure aromatique, tel que le benzène, toluène, xylène, l'éthylbenzène, ou un hydrocarbure aliphatique, tel que le cyclohexane, le pentane, l'heptane, le méthylcyclohexane ou une coupe aliphatique, ou un alcool, tel que le méthanol, éthanol, propanol ou isopropanol, butanol ou un isomère, ou un ester tel que l'acétate d'éthyle ou de méthyle, ou un éther tel que le tétrahydrofurane, diéthyléther, diisopropyl éther, le méthyltertiobutyléther ou un amide tel que le diméthylformamide, la N-méthyl pyrrolidone ou un carbonate tel que le diméthylcarbonate, le diéthylcarbonate, le carbonate d'éthylène, le carbonate de propylène ; ou encore un mélange de ces solvants.

De préférence le solvant comprend : le toluène, le xylène, le cyclohexane ou le méthylcyclohexane, ou des mélanges de ceux-ci.

La concentration en nitrile ester/acide insaturé dans le solvant peut varier de 1 à 70% et de préférence de 10 à 50% massique.

La quantité d'hydrogène utilisée est comprise de préférence dans la gamme de 3 à 200 moles pour 1 mole de nitrile ester/acide insaturé. La pression partielle en hydrogène H₂ est d'au moins 5 bars et au plus de 100 bars et de préférence au moins 15 bars et au plus 80 bars.

Type de réacteur :
Le nitrile ester/acide insaturé éventuellement mélangé avec un solvant et/ou de la base forme une phase liquide qui est mise en contact avec le catalyseur solide de toute manière convenable et on amène de l'hydrogène gazeux en contact avec le catalyseur solide et la phase liquide.

Dans le cas où un catalyseur finement divisé est utilisé, il peut être dispersé dans la phase liquide, et maintenu en dispersion par agitation. Cette agitation peut être réalisée dans un réacteur muni d'un agitateur, de préférence une turbine autoaspirante, par une recirculation munie d'un éjecteur gaz liquide (jet loop, Buss loop). [description http://www.airproducts.com/∼/media/downloads/h/hydrogen-support-microsite/hydrogen-support-increasing-productivity-in-slurry-hydrogenation-process.pdf]. Dans ce cas, la taille des particules de catalyseur est de préférence inférieure au mm typiquement comprise dans la gamme de 1 à 100 µm. On peut aussi utiliser un réacteur de type Continuous Oscillatory Baffled Reactor.

Un moyen facile de mise en œuvre de l'hydrogénation consiste à utiliser un lit de pastilles ou de granules de catalyseur solide. On peut faire passer la phase liquide sur le catalyseur, par écoulement dans le même sens ou à contre-courant d'un courant d'hydrogène. Le lit de catalyseur peut être complètement immergé dans la solution, ou le lit peut être utilisé sous forme de lit à ruissellement. Dans ce cas la taille des particules de catalyseur sera comprise dans la gamme de 1 à 10 mm et de préférence dans la gamme de 1 à 5 mm. On peut aussi utiliser un catalyseur sous la forme d'un monolithe structuré (par exemple sous la forme d'une mousse de céramique comme une mousse de SiC servant de support à la phase active).

La réaction peut être réalisée en discontinu par charges successives (batch) ou en continu.

Lors du chargement d'un nouveau lot de catalyseur dans un réacteur, celui-ci pourra subir un conditionnement avant d'être mis en contact avec le nitrile ester insaturé. Ce conditionnement pourra consister en des étapes d'inertage, des traitements thermiques et/ou l'injection d'hydrogène en concentration croissante.

La chaleur générée par la réaction peut être évacuée par tout moyen connu de l'homme de l'art : par des échangeurs de chaleur situés à l'intérieur du réacteur ou situés sur une boucle de recirculation externe au réacteur ou par circulation d'un fluide de refroidissement dans le cas de réacteur double enveloppe ou d'un réacteur multitubulaire ou réacteur à plaques ; ou encore par l'injection multi-étagée de réactif froid, comme de l'hydrogène froid.

Les quantités de catalyseur à utiliser varient selon l'activité du catalyseur et les conditions de réaction en particulier de la température et de la pression. Dans des opérations par charges successives, le ratio massique de catalyseur par rapport à la masse de nitrile ester insaturé peut varier de 0,01 à 1 et de préférence de 0,05 à 0,3. Le temps de réaction est compris dans la gamme de 1 h à 24 h et de préférence dans la gamme de 3h à 15h. Dans des opérations en continu, la vitesse spatiale liquide du catalyseur exprimée en kg/h de nitrile ester insaturé divisé par des kg de catalyseur peut varier de 0,01 à 10 h-1 et de préférence de 0,05 à 2 h-1.

C'est le choix du catalyseur décrit précédemment qui permet de limiter la production d'esters / acides alcanoïques par rapport à la quantité d'ester ou d'acide α,ω-amino-alcanoïque dans l'étape d'hydrogénation.

En hydrogénant selon le procédé décrit un nitrile ester/acide insaturé contenant moins de 0,3% d'acide ou ester alcanoïques, on obtient une composition contenant un ratio molaire entre les acides ou esters alcanoïques et l'ester ou acide α,ω-amino-alcanoïque compris dans la gamme de 0,001 à 0,4%.

Dans ces conditions, L'homme du métier sait adapter les conditions réactionnelles de l'hydrogénation (quantité de catalyseur, pression, température) de façon à maximiser la conversion du nitrile ester/acide insaturé et à obtenir une teneur en nitrile ester/acide saturé qui se forme intermédiairement dans la gamme de 0,0001% à 0,5% et de préférence de 0,0001% et 0,1% molaire par rapport à l'ester ou l'acide α,ω-amino-alcanoïque.

### Caractéristiques spécifiques au procédé B

La première étape d'hydrogénation est réalisée sur un catalyseur hétérogène qui contient au moins du palladium ou du rhodium ou du platine, ou leurs mélanges, de préférence au moins du palladium.

Le catalyseur selon l'invention contient typiquement de 0,1 à 10% de palladium ou rhodium et/ou platine, et de préférence de 0,2 à 5%.

Le palladium ou le rhodium ou le platine est avantageusement mis sur un support. Des exemples de supports appropriés sont la pierre ponce, le dioxyde de titane, le carbone, le charbon, le carbure de silicium (de préférence de forme beta SiC), l'alumine, la silice, un mélange de silice et d'alumine, la stéatite ou le carbonate de calcium.

Le catalyseur peut se présenter sous une forme quelconque telle que : poudre, sphère, grain, cylindre creux, trilobe et quadrilobe, ainsi que sous la forme de cylindres, extrudés ou compressés éventuellement en utilisant un agent de pastillage.

Avantageusement, la première réaction d'hydrogénation est conduite à une pression comprise dans la gamme de 1 à 90 bars, de préférence de 1 à 50 bars, de préférence de 2 à 20 bars, de préférence de 2 à 10 bars.

Avantageusement, la température est comprise dans la gamme de 10 à 100°C, de préférence de 20 à 100°C, de préférence dans la gamme de 20 à 50°C, de préférence de 30 à 50°C.

Cette étape est généralement réalisée sans solvant.

Selon un autre mode de réalisation, l'hydrogénation est réalisée en présence d'un solvant. Le solvant peut être un hydrocarbure aromatique, tel que le benzène, toluène, xylène, l'éthylbenzène ; ou un hydrocarbure aliphatique, tel que le cyclohexane, le pentane, l'heptane, le méthylcyclohexane ; ou une coupe aliphatique ; ou un alcool, tel que le méthanol, éthanol, propanol ou isopropanol, butanol ou un isomère, ou un ester tel que l'acétate d'éthyle ou de méthyle ; ou un éther tel que le tétrahydrofurane, diéthyléther, diisopropyl éther, le méthyltertiobutyléther ; ou un amide tel que le diméthylformamide, la N-méthyl pyrrolidone ; ou un carbonate tel que le diméthylcarbonate, le diéthylcarbonate, le carbonate d'éthylène, le carbonate de propylène ; et leurs mélanges.

De préférence le solvant est choisi parmi le toluène, le xylène, le cyclohexane ou le méthylcyclohexane, ou leurs mélanges.

La concentration en nitrile ester insaturé dans le solvant peut varier de 1 à 70%, de préférence de 10 à 50% massique.

La réaction peut être réalisée en discontinu par charges successives ou en continu. Les quantités de catalyseur à utiliser varient selon l'activité du catalyseur et les conditions de réaction en particulier de la température et de la pression. Dans des opérations par charges successives, le ratio massique de catalyseur par rapport à la masse de nitrile ester insaturé peut varier de 0,01 à 1 et de préférence de 0,05 à 0,3. Le temps de réaction est compris dans la gamme de 30 minutes à 24 h et de préférence de 1 h à 10h. Dans des opérations en continu, la vitesse spatiale liquide du catalyseur exprimée en kg/h de nitrile ester/acide divisé par kg de catalyseur peut varier de 0,01 à 10 h⁻¹, de préférence de 0,05 à 6 h⁻¹, de préférence de 0,05 à 4 h⁻¹, de préférence de 0,05 à 2 h⁻¹, de préférence de 0,2 à 2 h⁻¹, de préférence de 0,5 à 2 h⁻¹. On adaptera dans ces conditions : les quantités de catalyseur utilisé, la température, la pression et le temps de réaction ou la vitesse spatiale de façon à convertir plus de 70% du nitrile ester insaturé et de préférence plus de 90% et plus préférentiellement plus de 95%, Le produit formé est majoritairement le nitrile ester/acide saturé de formule NC-(CH2)(p+q+2)-CO2R, avec un taux de conversion compris dans la gamme de 70% à 99,5%, de préférence dans la gamme de 80 à 99%, de préférence dans la gamme de 90 à 99%, de préférence dans la gamme de 95 à 99%.

Avantageusement, la réaction de la première hydrogénation est réalisée en l'absence de base.

Le milieu réactionnel issu de la première hydrogénation est soumis à une deuxième hydrogénation, qui est réalisée en présence d'un catalyseur hétérogène qui contient du nickel et/ou du cobalt et/ou du ruthénium.

Le catalyseur selon l'invention contient typiquement 1% à 90% de nickel et de préférence 5% à 60%, et/ou 1% à 90% de cobalt et de préférence 5% à 60%, et/ou 0,1 à 10% de ruthénium et de préférence 1 à 5%. Le nickel et/ou le cobalt et/ou le ruthénium est de préférence dans sa forme réduite métallique. Le catalyseur introduit dans les réacteurs selon l'invention peut néanmoins contenir des oxydes de nickel, cobalt ou de ruthénium par exemple sous la forme d'une couche de passivation. En plus du nickel ou cobalt ou ruthénium, le catalyseur peut contenir d'autres métaux, tels que le fer, le nickel, le chrome, le manganèse, le rhodium, l'osmium, l'iridium, le platine, ou le palladium. Le catalyseur peut également être dopé par des alcalins tels le sodium, potassium, rubidium ou césium ou des alcalinoterreux tels que le magnésium, le calcium, le strontium ou le baryum. Le catalyseur peut se trouver à l'état finement divisé, par exemple sous la forme d'un catalyseur de type Raney. Il peut également se trouver sous la forme de granules, ou encore peut être déposé sur un support. Des exemples de supports appropriés sont la pierre ponce, le dioxyde de titane, le carbone, le charbon, le carbure de silicium (de préférence de forme beta SiC), l'alumine, la silice, un mélange de silice et d'alumine, la stéatite.

Le catalyseur sous forme de granule ou déposé sur un support peut se présenter sous une forme quelconque telle que : sphère, grain, cylindre creux, trilobe et quadrilobe, ainsi que sous la forme de cylindres, extrudés ou compressés éventuellement en utilisant un agent de pastillage.

Avantageusement, la seconde réaction d'hydrogénation est conduite à une pression comprise dans la gamme de 5 à 100 bars, de préférence de 20 à 80 bars. Avantageusement, la température est comprise dans la gamme de 50 à 150°C, de préférence de 80 à 110°C.

On ajoute une base pour diminuer la sélectivité en amine secondaire. La base peut être typiquement la soude, le méthoxyde de sodium, la potasse, le tertiobutylate de potassium ou l'ammoniac. De préférence on utilise de l'ammoniac. La quantité de base utilisée lors de la seconde réaction d'hydrogénation est telle que le ratio molaire base/nitrile ester ou acide insaturé à l'entrée de la première hydrogénation est typiquement compris dans la gamme de 0,1 à 4 et de préférence dans la gamme de 0,3 à 2,5.

SI la première hydrogénation a eu lieu en l'absence de solvant, la deuxième hydrogénation peut également être réalisée en l'absence de solvant. Elle peut aussi être réalisée en présence d'un solvant. Le solvant peut être un hydrocarbure aromatique, tel que le benzène, toluène, xylène, l'éthylbenzène ; ou un hydrocarbure aliphatique, tel que le cyclohexane, le pentane, l'heptane, le méthylcyclohexane ou une coupe aliphatique ; ou un alcool, tel que le méthanol, éthanol, propanol ou isopropanol, butanol ou un isomère ; ou un ester tel que l'acétate d'éthyle ou de méthyle ; ou un éther tel le tétrahydrofurane, diéthyléther, diisopropyl éther, le méthyltertiobutyléther ; ou un amide tel que le diméthylformamide, la N-méthyl pyrrolidone ; ou un carbonate tel que le diméthylcarbonate, le diéthylcarbonate, le carbonate d'éthylène, le carbonate de propylène ; et leurs mélanges.

De préférence le solvant est choisi parmi le toluène, le xylène, le cyclohexane ou le méthylcyclohexane, ou leurs mélanges.

La concentration en nitrile ester ou acide (insaturé et saturé) dans le solvant peut varier de 1 à 70% et de préférence de 10 à 50% massique.

Les quantités de catalyseur à utiliser dans la deuxième hydrogénation peuvent varier selon l'activité du catalyseur et les conditions de réaction en particulier de la température et de la pression. Dans des opérations par charges successives, le ratio massique de catalyseur par rapport à la masse de nitrile ester ou acide (saturé et insaturé) peut varier de 0,01 à 1 et de préférence de 0,05 à 0,3. Le temps de réaction est compris dans la gamme de 1 h à 24 h et de préférence de 3h à 15h. Dans des opérations en continu, la vitesse spatiale liquide du catalyseur exprimée en kg/h de nitrile ester divisé par des kg de catalyseur peut varier de 0,01 à 10 h⁻¹ et de préférence de 0,05 à 2 h⁻¹.

L'homme de l'art peut ainsi adapter les conditions réactionnelles de la deuxième hydrogénation, de préférence dans les gammes précitées (quantité de catalyseur, pression température), de façon à maximiser la conversion du nitrile ester saturé et à obtenir une teneur en nitrile ester saturé dans la gamme de 0,0001% à 0,5% et de préférence de 0,0001% à 0,1% molaire par rapport à l'ester ou l'acide α,ω-amino-alcanoïque.

### Caractéristiques communes aux procédé A et B

Avantageusement, on fait subir au mélange réactionnel issu de l'étape d'hydrogénation au moins une des étapes de purification suivantes :
- séparation du catalyseur par tout moyen de séparation solide liquide connu de l'homme du métier,
- séparation de la base : dans le cas de l'ammoniac par évaporation ou lavage aqueux, et dans le cas des autres bases par lavage aqueux ou équeutage dans un appareil à cours temps de séjour.
- séparation éventuelle du solvant par évaporation ou étêtage.
- séparation des lourds, et en particulier de l'amine secondaire formée lors de l'hydrogénation, par équeutage dans un appareil à cours temps de séjour sous vide à une température contrôlée.

Les appareils à court temps de séjour selon l'invention sont des évaporateurs à film, par exemple des évaporateurs à film tombant ou des évaporateurs couche mince où le film de liquide est créé mécaniquement ou par force centrifuge. L'évaporateur couche mince typique consiste en une surface d'échange thermique tubulaire équipée d'une enveloppe de chauffage externe et munie à l'intérieur d'un rotor à pales, avec racleurs fixe ou mobile. Les pales et racleurs ont pour fonction d'étaler le liquide sous la forme d'un film de faible épaisseur, afin de favoriser l'échange thermique et l'évaporation du produit, tout en limitant son temps de séjour et en limitant la formation et les dépôts d'oligomères et polymères encrassants.

Les vapeurs peuvent être condensées dans un condenseur externe ou interne. Avantageusement, les vapeurs peuvent être condensées dans un condenseur à l'intérieur de l'évaporateur de façon à pouvoir travailler sous vide encore plus poussé inférieur à 5 ou 1 mbar. On parle d'évaporateur couche mince à court trajet. Préférentiellement, la séparation des lourds est effectuée dans un évaporateur couche mince et de préférence un évaporateur couche mince à court trajet. L'étape de séparation des lourds est de préférence effectuée à une température inférieure à 160°C, de préférence inférieure à 140°C, de préférence inférieure à 130°C.

L'homme du métier sait adaptera la pression en fonction de la courbe de tension de vapeur de l'ester ou acide α,ω-amino-alcanoïque saturé, de façon à vaporiser cet ester ou acide dans l'appareil à court temps de séjour et récupérer dans les résidus de distillation les produits lourds et en particulier de l'amine secondaire formée lors de l'hydrogénation. Du fait de la faible volatilité des esters α,ω-amino-alcanoïque saturés de l'invention, il est nécessaire de travailler à vide poussé, généralement inférieur à 20 mbar, de préférence à une pression comprise dans la gamme de 0,5 à 20 mbar, de préférence 1 à 20 mbar, et de préférence inférieur à 10 mbar, de préférence à une pression de 0,5 à 5 mbar, de préférence de 1 à 5 mbar.

L'étape de séparation des lourds permet d'obtenir une composition qui comprend un ratio molaire entre l'amine secondaire de formule ROOC-(CH2)n-NH-(CH2)n-COOR et l'ester ou acide α,ω-amino-alcanoïque compris dans la gamme de 0,0001% à 1% et de préférence de 0,0001% à 0,5), ainsi qu'un ratio molaire initial entre le dimère ROOC-(CH2)n-NH-CO-(CH2)n-NH2 et l'ester ou acide α,ω-amino-alcanoïque compris dans la gamme de 0,0001% et 1%.

### UTILISATION DE LA COMPOSITION

La composition selon l'invention est utilisable comme matière première pour la fabrication de polyamide longue chaine.

Dans le cas d'une composition contenant de l'ester α,ω-amino-alcanoïque, la fabrication de polyamide peut notamment être effectuée en deux étapes comme décrites par exemple dans le document de brevet WO2015/071604, une première étape de réaction en présence d'eau à une première température, ayant pour fonction d'hydrolyser au moins partiellement la fonction ester, et une deuxième étape de réaction à une température plus élevée permettant d'obtenir le degré de polymérisation désiré.

La composition selon l'invention permet d'obtenir un polyamide avec un degré de polymérisation supérieur à 80, le degré de polymérisation d'un polyamide étant mesuré par RMN du proton, comme le double du ratio molaire entre les fonctions amide d'une part et la somme des fins de chaines (telles que fonctions amines, acide, alkyle et ester) d'autre part.

La composition selon l'invention permet aussi d'obtenir un polyamide dont la rhéologie à l'état fondu augmente plus faiblement à haute température, comme on peut le mesurer par exemple en suivant l'évolution de la viscosité à l'état fondu en rhéométrie plan-plan pendant 30 minutes à 270°C. On détermine la viscosité à l'état fondu à T0, puis après 30 minutes ainsi que le pourcentage de variation entre les deux. Une viscosité finale plus faible par exemple inférieure à 60000 et préférablement inférieure à 45000 Pa.s permet d'éviter les risques de blocage des vis de la machine de transformation en cas d'attente suite à un arrêt imprévu de la production.

En définitive, la solution de la présente invention présente plusieurs avantages essentiels par rapport à l'art antérieur :
La composition permet de produire des polyamides longue chaine à degré de polymérisation élevée, et qui sont stables à haute température.

Par rapport aux procédés d'hydrogénation de l'art antérieur, les procédés décrits permettent de limiter de manière contrôlée la quantité d'acide ou d'esters alcanoïque par rapport à l'amino acide / ester, ce qui permet au final de fabriquer des polymères plus facilement. Ils permettent également d'obtenir la composition selon l'invention avec un rendement élevé.

### EXEMPLES

### Exemple 1 : Préparation d'une composition selon l'invention

Du nitrile ester insaturé 10-cyano-9-décénoate de méthyle (NEI11) est préparé par réaction de 9-décénoate de méthyle avec l'acrylonitrile selon l'exemple 5 de WO2014/122410 suivi d'une évaporation du toluène et d'une distillation sous pression réduite de façon à obtenir du NEI11 contenant moins de 100 ppm molaire de décénoate de méthyle.

Dans un autoclave de 1 litre muni d'une turbine autoaspirante, on introduit 13,5 g de cobalt de Raney lavé au méthanol (ActiCatTM3100 de la société CatAlloy) et 90 g de nitrile ester insaturé NEI11 et 210 g de toluène. L'autoclave est purgé à l'azote, puis on introduit 4,3 g d'ammoniac (0,253 mol) et on additionne de l'hydrogène de façon à porter la pression à 60 bars. On chauffe à 90°C et on laisse réagir 10 heures sous agitation vigoureuse tout en maintenant la pression à 60 bars par addition d'hydrogène.

Le réacteur est alors refroidi à température ambiante, la pression est abaissée à pression atmosphérique. Le solide est séparé de la phase liquide et lavé par du toluène. Les phases liquides rassemblées sont évaporées à l'évaporateur rotatif à 30°C à une pression qui est abaissée progressivement jusqu'à 1 mbar. La phase liquide résiduelle est analysée par chromatographie gaz : elle contient 96% d'amine primaire, 2,4% d'amine secondaire, 0,1% de décanoate de méthyle, 0,1% de nitrile ester saturé 10-cyano-décanoate de méthyle (NE11) et 1% de toluène (% massiques).

Cette phase liquide résiduelle est alors distillée en continu dans un évaporateur à film raclé à court trajet de type KDL4 (UIC). L'évaporateur est maintenu à une pression de 0,7 mbar. Le liquide à distiller est alimenté à 150 g/h par le sommet de l'évaporateur et forme un film sur la surface d'échange chauffée à 120°C grâce à des racleurs à rouleaux. Les vapeurs sont condensées sur un échangeur central maintenu à 15°C.

On récupère une composition de 11-aminoundécanoate de méthyle (AE11) et de décanoate de méthyle (DM) dans un ratio molaire 99,9 / 0,1. La teneur massique en AE11 dans la composition est de 98,9%, celle en nitrile ester saturé 10-cyano-décanoate de méthyle (NE11) de 0,1%, celle en amine secondaire de formule (MeCO₂(CH₂)₁₀)₂NH 0,01% et celle en toluène 0,5%. Le rendement molaire en AE11 des étapes de réaction et de séparation est de 86%.

### Exemple 2 : Polymérisation de la composition obtenue à l'exemple 1

La composition d'AE11 et de DM de l'Exemple 1 (100 g), de l'eau (100g) et de l'acide phosphorique à 85% (0,06 g) sont introduits dans un réacteur à reflux total. On porte le mélange réactionnel à 110°C pendant 90 minutes sous agitation vigoureuse. Le milieu est ensuite lyophilisé (de sorte à éliminer l'eau et le méthanol), puis le lyophilisat est chauffé à 250°C sous balayage d'azote dans un réacteur en verre préalablement inerté à l'azote. Après 0h30 de chauffage, le polymère obtenu est refroidi puis analysé par RMN du proton. Le degré de polymérisation est calculé à partir de l'analyse RMN : c'est le double du ratio molaire entre les fonctions amide d'une part et la somme des fins de chaines (telles que fonctions amines, acide, alkyle et ester) d'autre part.

La stabilité de la rhéologie à l'état fondu est mesurée en suivant l'évolution de la viscosité à l'état fondu en rhéométrie plan-plan pendant 30 minutes à 270°C. On détermine la viscosité à l'état fondu à T0, puis après 30 minutes ainsi que le pourcentage de variation entre les deux.

Les résultats sont reportés au Tableau 1.

### Exemple 3 comparatif :

On reproduit l'Exemple 1 avec un catalyseur Nickel de Raney (W.R. Grace and Co. Raney® 2800 titrant à plus de 89% de Ni). Les résultats sont reportés au Tableau 1.

La phase liquide résiduelle après évaporation du toluène contient 95,5% d'amine primaire, 2,4% d'amine secondaire, 0,6% de décanoate de méthyle, 0,1% de nitrile ester saturé NE11 et 1% de toluène (% massiques).

Après distillation, on récupère une composition d'AE11 et de DM dans un ratio molaire 99,4 / 0,6. La teneur massique en AE11 dans la composition est de 98,4%, celle en nitrile ester saturé 10-cyano-décanoate de méthyle (NE11) de 0,1%, celle en amine secondaire de formule (MeCO₂(CH₂)₁₀)₂NH 0,02% et celle de toluène de 0,5%.

### Exemple 4 : Polymérisation de la composition obtenue à l'exemple 3

La composition d'AE11 et de DM de l'exemple 3 est polymérisée de la même façon qu'à l'Exemple 2 et on mesure le degré de polymérisation et la stabilité de la rhéologie à l'état fondu. Les résultats sont reportés au Tableau 1.

On constate que le degré de polymérisation est beaucoup plus faible qu'à l'Exemple 2.

### Exemple 5 comparatif :

Dans un réacteur en verre de 2 litres muni d'un réfrigérant, on introduit 302 g d'acide 11-aminoundécanoïque commercial (Sigma-Aldrich) dans 1 litre de méthanol. À 60°C, on injecte 30 normaux litres par heure d'HCl anhydre pendant 3 heures. Le mélange réactionnel est alors évaporé à l'évaporateur rotatif sous 0,2 bar à 35 puis 45°C. Le solide est repris dans 2 litres d'éther éthylique puis lavé à 5°C par 380 ml de soude aqueuse à 20%. Après décantation et mise de côté de la phase aqueuse, la phase organique est lavée successivement par 200 ml de soude aqueuse à 1% puis par 4 fois 200 ml d'eau déminéralisée. La première phase aqueuse est réextraite avec 500 ml d'éther éthylique et la phase éthérée est lavée successivement avec les phases aqueuses obtenues précédemment. On renouvelle l'opération d'extraction à nouveau avec 500 ml d'éther. Les phases organiques rassemblées sont séchées sur sulfate de magnésium puis évaporées à l'évaporateur rotatif à 20-30°C en abaissant progressivement la pression jusqu'à 1 mbar.

La phase liquide récupérée est distillé en continu dans un évaporateur à film raclé à court trajet de la même façon qu'à l'Exemple 1. On obtient un liquide composé à plus de 99,5% d'AE11 et contenant moins de 1 ppm d'acide ou d'esters alcanoïques.

### Exemple 6 : Polymérisation de la composition obtenue à l'exemple 3

La composition d'AE11 contenant moins de 1 ppm d'acide ou d'esters alcanoïques, produite à l'Exemple 5, est polymérisée de la même façon qu'à l'Exemple 2 et on mesure le degré de polymérisation et la stabilité de la rhéologie à l'état fondu du polymère (polyamide 11).

Les résultats sont reportés au Tableau 1.

On constate que le polymère est beaucoup moins stable en température que celui de l'Exemple 2.

### Exemple 7 : Préparation d'une composition selon l'invention

Du nitrile ester insaturé 10-cyano-9-décénoatede méthyle (NEI11) contenant moins de 100 ppm molaire de décénoate de méthyle est préparé de la même façon qu'à l'Exemple 1.

Du carbure de silicium (Sicat CTS-17 de surface BET 24 m²/g et composé à plus de 99% de β-SiC et moins de 0,5% de Fe) est broyé et tamisé à la granulométrie de 0,1-0,2 mm, puis séché à l'étuve à 120°C pendant 2 heures. Une fraction de 20 g de la poudre obtenue est imprégnée au volume poreux à humidité naissance (« incipient wetness impregnation » en anglais) avec 11,4 ml d'une solution aqueuse de nitrosyl nitrate de ruthénium III à 0,08 g d'ion Ru par ml. Le solide est alors calciné sous flux d'air dans un tube chauffé à 2°C/min jusqu'à 270°C et maintenu à cette température pendant 4 heures.

Une partie du solide obtenu (10 g) est placée dans un réacteur lit fixe. Le catalyseur est réduit par un traitement sous hydrogène à 240°C, puis on injecte 4,8 normaux litres par heure d'un mélange d'ammoniac à 3% volumique dans de l'hydrogène et 10 ml/h d'une solution de 10-cyano-9-décénoate de méthyle à 30% massique dans le toluène. Le réacteur est maintenu à une température de 80°C et une vanne de détente assure une pression de 50 bar à l'intérieur du réacteur. La phase liquide en sortie réacteur est récupérée sur une durée de 70 heures. Après évaporation à l'évaporateur rotatif à 30°C à une pression qui est abaissée progressivement jusqu'à 1 mbar, on obtient une phase liquide résiduelle qui est analysée par chromatographie gaz : elle contient 95% d'amine primaire, 3,6% d'amine secondaire, 0,27% de décanoate de méthyle, 0,1% de nitrile ester saturé NE11 et 1% de toluène (% massiques).

Cette phase liquide résiduelle est alors distillée en continu dans un évaporateur à film raclé à court trajet de la même façon qu'à l'Exemple 1.

On récupère une composition de 11-aminoundécanoate de méthyle (AE11) et de décanoate de méthyle (DM) dans un ratio molaire 99,73 / 0,27. La teneur massique en AE11 dans la composition est de 98,9%, celle en nitrile ester saturé 10-cyano-décanoate de méthyle (NE11) de 0,1%, celle en amine secondaire de formule (MeCO₂(CH₂)₁₀)₂NH 0,02% et la celle en toluène 0,5%. Le rendement molaire en AE11 des étapes de réaction et de séparation est de 85%.

### Exemple 8 : Polymérisation de la composition obtenue à l'exemple 7

L'AE11 produit à l'Exemple 7 est polymérisé de la même façon qu'à l'Exemple 2 et on mesure le degré de polymérisation et la stabilité de la rhéologie à l'état fondu.

Le résultat est reporté au Tableau 1.

### Exemple 9 : Préparation d'une composition selon l'invention

Du nitrile ester insaturé 10-cyano-9-décénoate de méthyle (NEI11) contenant moins de 100 ppm molaire de décénoate de méthyle est préparé de la même façon qu'à l'Exemple 1.

Du carbure de silicium (Sicat CTS-17 de surface BET 24 m²/g et composé à plus de 99% de β-SiC et moins de 0,5% de Fe) est broyé et tamisé à la granulométrie de 0,1-0,2 mm, puis séché à l'étuve à 120°C pendant 2 heures. Une fraction de 20 g de la poudre obtenue est imprégnée au volume poreux à humidité naissance (« incipient wetness impregnation » en anglais) avec 1,14 ml d'une solution aqueuse de nitrate de cobalt II hexahydrate à 0,176 g d'ion Co par ml. Le solide est alors calciné sous flux d'air dans un tube chauffé 5°C/min jusqu'à 400°C et maintenu à cette température pendant 4 heures. Il est ensuite réduit sous flux d'hydrogène à 350°C pendant 4 heures, puis passivé à température ambiant sous flux d'air appauvri à 0,1% d'oxygène puis à 1% d'oxygène.

Une partie du solide obtenu (7 g) est placée dans un réacteur lit fixe. Le catalyseur est réduit par un traitement sous hydrogène à 240°C, puis on injecte 4,8 normaux litres par heure d'un mélange d'ammoniac à 3% volumique dans de l'hydrogène et 10 ml/h d'une solution de 10-cyano-9-décénoate de méthyle à 30% massique dans le toluène. Le réacteur est maintenu à une température de 80°C et une vanne de détente assure une pression de 50 bar à l'intérieur du réacteur. La phase liquide en sortie de réacteur est récupérée sur une durée de 70 heures. Après évaporation à l'évaporateur rotatif à 30°C à une pression qui est abaissée progressivement jusqu'à 1 mbar, on obtient une phase liquide résiduelle qui est analysée par chromatographie gaz : elle contient 90% d'amine primaire, 5% d'amine secondaire, 0,12% de décanoate de méthyle, 0,1% de nitrile ester saturé NE11 et 1% de toluène (% massiques).

Cette phase liquide résiduelle est alors distillée en continu dans un évaporateur à film raclé à court trajet de la même façon qu'à l'Exemple 1.

On récupère une composition de 11-aminoundécanoate de méthyle (AE11) et de décanoate de méthyle (DM) dans un ratio molaire 99,88 / 0,12.

### Exemple 10 : Polymérisation de la composition obtenue à l'exemple 9

L'AE11 produit à l'Exemple 9 est polymérisé de la même façon qu'à l'Exemple 2 et on mesure le degré de polymérisation et la stabilité de la rhéologie à l'état fondu.

Les résultats sont reportés au Tableau 1.

### Exemple 11 : Préparation d'une composition selon l'invention

Du nitrile ester insaturé 10-cyano-9-décénoate de méthyle (NEI11) contenant moins de 100 ppm molaire de décénoate de méthyle est préparé de la même façon qu'à l'Exemple 1.

Dans un autoclave de 1 litre muni d'une turbine autoaspirante, on introduit 10 g de palladium à 2% sur charbon réduit en poudre (BASF Italie référence 51678063-5254) et 90 g de nitrile ester insaturé NEI11 et 210 g de toluène. L'autoclave est purgé à l'azote, puis on additionne de l'hydrogène de façon à maintenir une pression de 5 bar. Après 8 heures sous agitation vigoureuse à 30-40°C, le milieu réactionnel est détendu puis filtré. Une analyse par chromatographie gaz montre que le NEI11 a été converti à 97%.

La phase liquide est introduite à nouveau dans l'autoclave contenant 13,5 g de Nickel de Raney lavé au méthanol (W.R. Grace and Co. Raney® 2800 titrant à plus de 89% de Ni). L'autoclave est purgé à l'azote, puis on introduit 4,3 g d'ammoniac (0,253 mol) et on additionne de l'hydrogène de façon à porter la pression à 60 bars. On chauffe à 90°C et on laisse réagir 12 heures sous agitation vigoureuse tout en maintenant la pression à 60 bars par addition d'hydrogène.

Le réacteur est alors refroidi à température ambiante, la pression est abaissée à pression atmosphérique. Le solide est séparé de la phase liquide et lavé par du toluène. Les phases liquides rassemblées sont évaporées à l'évaporateur rotatif à 30°C à une pression qui est abaissée progressivement jusqu'à 1 mbar. La phase liquide résiduelle est analysée par chromatographie gaz : elle contient 96% d'amine primaire, 2,3% d'amine secondaire, 0,03% de décanoate de méthyle, 0,03% de nitrile ester saturé NE11 et 1% de toluène (% massiques).

Cette phase liquide résiduelle est alors distillée en continu dans un évaporateur à film raclé à court trajet de la même façon qu'à l'Exemple 1.

On récupère une composition de 11-aminoundécanoate de méthyle (AE11) et de décanoate de méthyle (DM) dans un ratio molaire 99,97 / 0,03.

### Exemple 12 : Polymérisation de la composition obtenue à l'exemple 11

L'AE11 produit à l'Exemple 11 est polymérisé de la même façon qu'à l'Exemple 2 et on mesure le degré de polymérisation et la stabilité de la rhéologie à l'état fondu.

Les résultats sont reportés au Tableau 1.

### Exemple 13 :

Une solution à 2% massique d'acide heptanoïque dans du méthanol (5 grammes) est pulvérisée sur 99,9 g d'acide 11-aminoundécanoïque commercial en poudre (Sigma-Aldrich) sous agitation vigoureuse. Le méthanol est ensuite évaporé sous pression réduite. On obtient un mélange d'acide 11-aminoundécanoïque à 0,15% molaire d'acide heptanoïque.

### Exemple 14 : Polymérisation de la composition obtenue à l'exemple 13

La composition d'acide 11-aminoundécanoïque et d'acide heptanoïque de l'exemple 13 (100 g), additionnée d'acide phosphorique à 85% (0,06 g) est introduite dans un réacteur en verre préalablement inerté à l'azote. On porte le mélange réactionnel sous balayage d'azote à 250°C sous agitation vigoureuse. Après 0h30 de chauffage, le polymère obtenu est refroidi.

On mesure le degré de polymérisation et la stabilité de la rhéologie à l'état fondu de la même façon qu'à l'exemple 2.

Les résultats sont reportés au Tableau 1.

**Tableau 1**

| **Exemple** | Ratio molaire Acide/ester alcanoïque sur Acide/ester amino alcanoïque | Degré de polymérisation | Viscosité à l'état fondu à 270°C (Pa.s) | | |
|---|---|---|---|---|---|
| | | | Initiale | Après 30 minutes | Variation en 30 minutes |
| **Exemple 2** | 0,1% | 140 | 16800 | 44300 | +164% |
| **Exemple 4 comparatif** | 0,6% | 70 | 620 | 1120 | +80% |
| **Exemple 6 comparatif** | <1 ppm | 150 | 21200 | 75200 | +254% |
| **Exemple 8** | 0,27% | 120 | 4090 | 10200 | +149% |
| **Exemple 10** | 0,12% | 135 | 15900 | 41300 | +160% |
| **Exemple 12** | 0,03% | 145 | 19100 | 55700 | +192% |
| **Exemple 14** | 0,15% | 145 | 17200 | 45800 | +166% |

## Revendications

1. Composition comprenant au moins un ester ou acide α,ω-amino-alcanoïque et au moins un acide ou ester alcanoïque, **caractérisée en ce que** :
- l'ester ou acide α,ω-amino-alcanoïque a pour formule H₂N-(CH₂)ₙ-COOR avec n=9 à 13 et R est un groupement alkyle ou un hydrogène,
- l'acide ou ester alcanoïque a pour formule R₁-COOR₂ avec R₁ un groupement alkyle linéaire ou ramifié de formule CₘH₂ₘ₊₁ ou CₘH₂ₘ₋₁ ou CₘH₂ₘ₋₃ où m=6 à 20 et R₂ est un groupement alkyle ou un hydrogène,
- le ratio molaire entre acide(s) ou ester(s) alcanoïques et ester(s) ou acide(s) α,ω-amino-alcanoïque est compris dans la gamme de 0,001 à 0,4%.

2. Composition selon la revendication 1, dans laquelle n=10 ou 11, et R est un groupement méthyle ou éthyle ou butyle ou un hydrogène.

3. Composition selon l'une quelconque des revendications 1 ou 2, dans laquelle R₁ est un groupement alkyle linéaire de composition Cₙ₋₁H₂ₙ₋₁ et R₂ est un groupement méthyle ou éthyle ou un hydrogène.

4. Composition selon l'une quelconque des revendications 1 à 3, **caractérisée en ce qu'**elle comprend en outre un nitrile acide ou un nitrile ester de formule NC-(CH₂)(ₙ₋₁)-COOR, dans laquelle le ratio molaire de nitrile par rapport à l'ester ou acide α,ω-amino-alcanoïque est compris dans la gamme de 0,0001% à 0,5%, de préférence compris dans la gamme de 0,0001% à 0,1%, de préférence compris dans la gamme de 0,0001% à 0,05%.

5. Composition selon l'une quelconque des revendications 1 à 4, **caractérisée en ce qu'**elle comprend en outre une amine secondaire de formule ROOC-(CH2)ₙ-NH-(CH₂)ₙ-COOR, dans laquelle le ratio molaire par rapport à l'ester ou acide α,ω-amino-alcanoïque est compris dans la gamme de 0,0001% à 1%, de préférence de 0,0001% à 0,5%.

6. Composition selon l'une quelconque des revendications 1 à 5, **caractérisée en ce qu'**elle comprend en outre du dimère ROOC-(CH₂)ₙ-NH-CO-(CH₂)ₙ-NH₂, le ratio molaire en dimère par rapport à l'ester ou acide α,ω-amino-alcanoïque étant compris dans la gamme de 0,0001% à 5% et de préférence compris dans la gamme de 0,0001% à 1%.

7. Composition selon l'une quelconque des revendications 1 à 6, **caractérisée en ce que** la teneur en ester ou acide α,ω-amino-alcanoïque est supérieure à 10%, de préférence supérieure à 50%, de préférence supérieure à 90% et plus préférablement supérieure à 98%, en poids sur le poids total de la composition.

8. Polymère, notamment polyamide, obtenu par polymérisation de la composition de l'une quelconque des revendications 1 à 7, **caractérisé en ce que** son degré de polymérisation est supérieur à 80.

9. Procédé A de préparation de la composition selon l'une quelconque des revendications 1 à 7, comprenant l'hydrogénation d'un nitrile acide/ester aliphatique insaturé en présence d'hydrogène, **caractérisé en ce que** :
- le nitrile acide/ester aliphatique insaturé contient en outre de 0,001 à 0,3%, de préférence de 0,001 à 0,1%, d'acide/ester alcanoïque ; et
- l'hydrogénation est réalisée en présence :
i. d'un catalyseur hétérogène qui comprend de 1 à 90%, de préférence de 5 à 60%, de Co et/ou de 0,1 à 10%, de préférence de 1 à 5%, de Ru ; et
ii. d'une base, le ratio molaire base/nitrile est compris dans la gamme de 0,1 à 4, de préférence dans la gamme de 0,3 à 2,5.

10. Procédé selon la revendication 9, dans lequel la réaction est réalisée en continu.

11. Procédé selon l'une quelconque des revendications 9, dans lequel la réaction est réalisée en discontinu par charges successives.

12. Procédé selon l'une quelconque des revendications 9 à 11, dans lequel l'hydrogénation est conduite à une pression comprise dans la gamme allant de 5 à 100 bars, de préférence de 20 à 80 bars, et à une température comprise dans la gamme allant de 50 à 150°C, de préférence de 80 à 110°C.

13. Procédé selon l'une quelconque des revendications 9 à 12, dans lequel la quantité d'hydrogène utilisée est comprise dans la gamme de 3 à 200 moles pour 1 mole de nitrile acide/ester insaturé, la pression partielle en hydrogène étant comprise dans la gamme de 5 bars à 100 bars, de préférence dans la gamme de 15 bars à 80 bars.

14. Procédé B de préparation de la composition selon l'une quelconque des revendications 1 à 7, comprenant deux étapes d'hydrogénation successives à partir d'un nitrile acide/ester aliphatique insaturé, **caractérisé en ce que** :
- le nitrile acide/ester aliphatique insaturé contient comme impureté de 0,001 à 0,3%, de préférence de 0,001 à 0,1%, d'acide/ester alcanoïque ; et
- la première étape d'hydrogénation est réalisée en présence d'un catalyseur hétérogène comprenant de 0,1 à 10%, de préférence de 0,2 à 5%, de palladium et/ou du rhodium et/ou du platine, de préférence du palladium, de sorte que le nitrile acide/ester aliphatique insaturé est transformé en nitrile acide/ester aliphatique saturé de formule NC-(CH₂)_{(p+q+2)}-CO2R selon un taux de conversion compris dans la gamme de 70% à 99,5%, de préférence dans la gamme de 80 à 99%, de préférence dans la gamme de 90 à 99%, de préférence dans la gamme de 95 à 99% ; et
- la deuxième étape d'hydrogénation est réalisée en présence d'un catalyseur hétérogène qui comprend de 1% à 90%, de préférence de 5 à 60% de Ni et/ou de 1 à 90%, de préférence de 5 à 60% de Co et/ou de 0,1 à 10%, de préférence de 1 à 5%, de Ru, de sorte que le nitrile acide/ester aliphatique saturé est transformé en amino acide/ester saturé.

15. Procédé selon la revendication 14, dans lequel la première étape d'hydrogénation est conduite à une pression comprise dans la gamme allant de 1 à 90 bars, de préférence de 1 à 50 bars, de préférence de 2 à 20 bars, de préférence de 2 à 10 bars, et à une température comprise dans la gamme allant de 10 à 100°C, de préférence de 20 à 100°C, de préférence de 20 à 50°C, de préférence de 30 à 50°C.

16. Procédé selon l'une des revendications 14 ou 15, dans lequel la réaction de la première hydrogénation est réalisée en continu.

17. Procédé selon l'une quelconque des revendications 14 à 16, dans lequel la réaction de la première hydrogénation est réalisée en discontinu par charges successives.

18. Procédé selon l'une quelconque des revendications 14 à 17, dans lequel la seconde réaction d'hydrogénation est conduite à une pression comprise dans la gamme de 5 à 100 bars, de préférence 20 à 80 bars ; et à une température comprise dans la gamme de 50 à 150°C, de préférence de 80 à 110°C.

19. Procédé selon l'une quelconque des revendications 14 à 18, dans lequel la deuxième hydrogénation est réalisée en continu.

20. Procédé selon l'une quelconque des revendications 14 à 18, dans lequel la deuxième hydrogénation est réalisée en discontinu par charges successives.

21. Procédé selon l'une quelconque des revendications 9 à 20, dans lequel le milieu réactionnel issu de l'hydrogénation est soumis à au moins l'une des étapes de purification suivantes :
- séparation du catalyseur par tout moyen de séparation solide liquide ;
- séparation de la base : dans le cas de l'ammoniac par évaporation ou lavage aqueux et dans le cas des autres bases par lavage aqueux ou équeutage dans un appareil à cours temps de séjour.
- séparation du solvant éventuel par évaporation ou étêtage.

22. Procédé selon l'une quelconque des revendications 9 à 21, **caractérisé en ce qu'**il comprend en outre une étape de séparation de lourds, en particulier d'amine secondaire formée lors de l'hydrogénation, par équeutage dans un appareil à cours temps de séjour sous vide, à une pression comprise dans la gamme de 0,5 à 20 mbar, de préférence de 1 à 20 mbar, de préférence de 1 à 5 mbar, à une température contrôlée, inférieure à 160°C, de préférence inférieure à 140°C et de préférence inférieure à 130°C.
